# EUROPEAN PATENT SPECIFICATION

(11) **EP 1 368 287 B1**
(45) Date of publication and mention of the grant of the patent: **26.03.2008**
(21) Application number: 02710548.5
(22) Date of filing: 18.01.2002
(51) Int. Cl.: C07B 37/04, C07C 67/343, C07C 69/738, C07D 213/55, C07C 253/30, C07C 49/258, C07C 255/56, C07C 205/45, C07C 49/255, C07C 49/223, C07C 49/217, C07C 49/235, C07C 225/22, C07C 25/24, C07C 43/215

(54) **PROCESS FOR A HOMOGENEOUSLY CATALYZED C-C COUPLING REACTION**
VERFAHREN ZU EINER HOMOGEN KATALISIERTEN CC-KUPPLUNGSREAKTION
METHODE DE REALISATION D'UNE REACTION DE LIAISON C-C PAR CATALYSATION HOMOGENE

(30) Priority: 18.01.2001 NL 1017138
(43) Date of publication of application: 10.12.2003
(73) Proprietor: DSM IP Assets B.V., 6411 TE Heerlen (NL)
(72) Inventor: VRIES DE, Andreas, Hendrikus, Maria, NL-6211 SW Maastricht (NL); VRIES DE, Johannes, Gerardus, NL-6228 GZ Maastricht (NL)
(74) Representative: Scheltus, Irma
(86) International application number: PCT/NL2002/000037
(87) International publication number: WO 2002/057199

(56) References cited:
- DE-A- 19 712 388
- WALLOW T I ET AL: "HIGHLY EFFICIENT AND ACCELERATED SUZUKI ARYL COUPLINGS MEDIATED BY PHOSPHINE-FREE PALLADIUM SOURCES" JOURNAL OF ORGANIC CHEMISTRY, AMERICAN CHEMICAL SOCIETY. EASTON, US, vol. 59, no. 17, 1994, pages 5034-5037, XP002030761 ISSN: 0022-3263
- REETZ M T ET AL: "A New Catalyst System for the Heck Reaction of Unreactive Aryl Halides" ANGEWANDTE CHEMIE. INTERNATIONAL EDITION, VERLAG CHEMIE. WEINHEIM, DE, vol. 37, no. 4, 1998, pages 481-483, XP002160618 ISSN: 0570-0833
- REETZ, M.T., WESTERMANN, E.: "Phosphane free palladium catalyzed coupling reactions: the decisive role of Pd nanoparticles" ANGEW. CHEM. INT. ED., vol. 39, no. 1, 3 January 2000 (2000-01-03), pages 165-8, XP002179534
- REETZ M T ET AL: "Suzuki and Heck Reactions Catalyzed by Preformed Palladium Clusters and Palladium/Nickel Bimetallic Clusters" TETRAHEDRON LETTERS, ELSEVIER SCIENCE PUBLISHERS, AMSTERDAM, NL, vol. 37, no. 26, 24 June 1996 (1996-06-24), pages 4499-4502, XP004029050 ISSN: 0040-4039
- REETZ M T ET AL: "A Highly Active Phosphine-free Catalyst System for Heck Reactions of Aryl Bromides" TETRAHEDRON LETTERS, ELSEVIER SCIENCE PUBLISHERS, AMSTERDAM, NL, vol. 39, no. 46, 12 November 1998 (1998-11-12), pages 8449-8452, XP004139440 ISSN: 0040-4039
- MEHNERT ET AL.: "Palladium-grafted mesoporous MCM-41 material as heterogeneous catalyst for Heck reactions" CHEM. COMMUN., 1997, pages 2215-6, XP002179535
- BELLER M ET AL: "First palladium-catalyzed Heck reactions with efficient colloidal catalyst systems" JOURNAL OF ORGANOMETALLIC CHEMISTRY, ELSEVIER-SEQUOIA S.A. LAUSANNE, CH, vol. 520, no. 1, 9 August 1996 (1996-08-09), pages 257-259, XP004036483 ISSN: 0022-328X
- JEFFERY: "Highly stereospecific palladium-catalyzed vinylation of vinylic halides under solid-liquid phase transfer conditions" TETRAHEDRON LETTERS, vol. 26, no. 22, 1985, pages 2667-70, XP002179536
- BUMAGIN ET AL.: "Palladium-catalyzed phenylation and vinylaton" BULL. ACAD. SCI. USSR DIV. CHEM. SCI. (ENGL. TRANSL.), vol. 39, no. 11, 1990, page 2426 XP002179537
- JOHANNES G. DE VRIES ET AL.: "The Power of High-Throughput Experimentation in Homogeneous Catalysis Research for Fine Chemicals" EUR. J. ORG. CHEM., 2003, pages 799-811,
- ANDR¹ H. M. DE VRIES ET AL.: "Homeopathic Ligand-Free Palladium as a Catalyst in the Heck Reaction. A Comparison with a Palladacycle" ORGANIC LETTERS, vol. 5, no. 18, 2003, pages 3285-3288,
- ASAF ALIMARDANOV ET AL.: "Use of "Homeopathic" Ligand-Free Palladium as Catalyst for Aryl-Aryl Coupling Reactions" ADV. SYNTH. CATAL., vol. 346, 2004, pages 1812-1817,

## Description

The invention relates to a process for carrying out a homogeneously catalyzed C-C coupling reaction between an optionally substituted (hetero)aromatic bromide compound and a second reactant, which is chosen from the group of olefins in which at least one of the substituents on the olefinic Sp² carbon atoms is a hydrogen atom, and the group of organoboron compounds with the formula Ar-B(OR¹)-OR², where Ar stands for an optionally substituted (hetero)aryl group and R¹ and R² each independently represent H or an alkyl group or, together with the O-atoms to which they are bound and the B-atom, form a ring with 2-5 C-atoms, in the presence of a palladium catalyst, an aprotic dipolar solvent and a base.

Homogeneously catalyzed C-C coupling reactions are known from the literature. Examples of such reactions are arylations of olefins and aryl-aryl-couplings. For example, in "A Highly Active Phosphine-free Catalyst System for Heck of Aryl Bromides", by M.T. Reetz et all, Tetrahedron letters, 39 (1998) 8449-8452, reactive and selective catalyst systems consisting of palladiums salts such as Pd(OAc)₂ or PdCl₂ (PhCN)₂ in the presence of N,N-dimethyl glycine (DMG) for Heck reactions of aryl bromides with defines are described, and in "Metal-catalyzed Cross-coupling reactions", F. Diederich and P.J. Stang Eds., Wiley-VCH, Weinheim, 1998, Chapters 2 and 3, examples of arylations of olefines and aryl-aryl-couplings are described. In practice the molar ratio between the quantity of palladium and the optionally substituted (hetero)aromatic bromide compound in these C-C coupling reactions usually lies between 1 and 3 mol% and one or more ligands are present, which are used to prevent the precipitation of metallic palladium. In the framework of the invention ligand is therefore understood to be a compound that is used in order to keep the palladium in solution. Examples of ligands used in practice are given for example in "Metal-catalyzed Cross-coupling reactions", F. Diederich and P.J. Stang Eds., Wiley-VCH, Weinheim, 1998, Chapters 2 and 3. Commonly used ligands are phosphines.

In W.A. Herrmann, V.P.W. Bohm and C.-P. Reisinger, J. Organomet.Chem.,576 (1999), p. 23-41, the recent development of palladacycles is described. Palladacycles are used as palladium catalysts in C-C-coupling reactions at relatively low ratios between the quantity of palladium and the optionally substituted (hetero)aromatic bromide compound, namely between 0.0001 and 2 mol%. Palladacyles are prepared from a palladium salt and ligands, for example o-tolylphosphine, tris(2,4-di-tert-butyl)phosphite or a benzylic thioether, and should thus be regarded as a combination of catalyst and ligand.

A disadvantage of the above processes is that they are expensive. In many cases the ligands used are expensive and may hinder the work up of the reaction mixture after the C-C-coupling reaction. In addition, as a rule large quantities of catalyst are used relative to the quantity of substrate, which increases the catalyst costs and may hinder the work up of the reaction mixture. For the preparation of palladacycles in particular laborious methods are usually necessary.

The aim of the invention is to provide a cheap, simple and commercially attractive process for homogeneously catalyzed C-C coupling reactions with an optionally substituted (hetero)aromatic bromide compound.

This is accomplished according to the invention by using a palladium salt without a ligand as a palladium catalyst and carrying out the reaction at a ratio of the quantity of palladium present in the palladium salt to the optionally substituted (hetero)aromatic bromide compound between 0.01 and 0.1 mol%.

In the framework of the invention the quantity of palladium present in the palladium salt is understood to be the quantity of palladium ions present in the total amount of palladium salt added.

Surprisingly, it has been found that with the aid of this simple process, in which no ligand and only very little palladium salt is used, such favourable results are obtained that a cheap process can be developed that in practice is easy to scale up and therefore is pre-eminently suitable for commercial applications. Surprisingly, it has been found that, despite the absence of a ligand, no or hardly any precipitation of the palladium catalyst takes place during the reaction. Surprisingly, the activity of the palladium catalyst appears to be so high that the method according to the invention can be suitably applied in C-C coupling reactions with optionally substituted (hetero)aromatic bromide compounds, which are relatively cheap. This is particularly surprising because such bromide compounds are known to be much less reactive than the corresponding, much more expensive iodine compounds. In the process according to the invention, for instance a conversion of more than 80%, preferably more than 90% in particular more than 95% can be achieved in less than 24 hours, preferably in less than 15 hours, more preferably in less than 5 hours and often even in less than 2 hours.

In the process according to the invention an optionally substituted (hetero)aromatic bromide compound is coupled with an olefin or a organoboron compound.

Suitable examples of (hetero)aromatic groups from which the bromide compound has been derived are phenyl, naphthyl, pyridyl, pyrrolyl, quinolyl, isoquinolyl, furyl, thienyl, benzofuryl, indenyl, pyrimidinile, pyrazolyl and imidazolyl. The (hetero)aromatic group can optionally be substituted with one or more substituents, in principle all substituents which are inert under the given reaction conditions. Suitable examples of such substituents are an alkyl group with for example 1 to 20 carbon atoms, for example a methyl, ethyl, isobutyl or trifluoromethyl group; an alkenyl group with for example 2 to 20 carbon atoms; a (hetero)aryl group with for example 1 to 50 carbon atoms; a carboxyl group; an alkyl or aryl carboxylate group with for example 2 to 50 carbon atoms; a formyl group; an alkanoyl or aroyl group with for example 2 to 50 carbon atoms; a carbamoyl group; an N-substituted alkyl or aryl carbamoyl group with for example 2 to 50 carbon atoms; an amino group; an N-substituted alkyl or arylamino group with for example 1 to 50 carbon atoms; a formamido group; an alkyl or aryl amido group with for example 2 to 50 carbon atoms; a hydroxy group; an alkoxy or aryloxy group with for example 1 to 50 carbon atoms; cyano; nitro; halogen and an alkyl or arylthio group with for example 1 to 50 carbon atoms. Preferred examples of (hetero)aromatic groups are substituted phenyl groups and fused aromatic groups, for instance groups derived from naphthyl, for example a biphenyl, 4-fluoro-phenyl, naphthyl or 6-chloro-naphthyl group.

In particular the process according to the invention surprisingly also appeared to be applicable to compounds of which the optionally substituted (hetero)aromatic bromide compound contains at least one hetero atom chosen from N, O and S. Suitable examples of such compounds are bromoacetophenones, for example 4-bromoacetophenone; bromopyridines, for example 3-bromopyridine; bromobenzonitriles, for example 2-bromobenzonitrile or 4-bromobenzonitrile; bromobenzaldehydes, for example 2-bromobenzaldehyde or 4-bromobenzaldehyde; bromonitrobenzenes, for example 4-bromonitrobenzene; 2-bromine-6-methoxynaphthalene and bromoanisoles, for example 4-bromoanisole. These compounds have a good activity in the method according to the invention. This is surprising, because the optionally substituted (hetero)aromatic bromide compound is present in a very large excess relative to the palladium catalyst in the method according to the invention and it was expected that this compound would act as a catalyst poison due to the presence of one or more hetero atoms.

In an embodiment of the invention as second reactant an olefin is used with formula (1)

R³R⁴C=CHR⁵ (1)

in which R³, R⁴ and R⁵ can each be chosen independently of each other in function of the desired final product and can represent both electron-donating, electron-withdrawing and electron-neutral groups. Suitable choices for R³, R⁴ and R⁵ are: hydrogen; an alkyl group with for example 1 to 20 carbon atoms, for example a methyl, ethyl, or trifluoromethyl group; an alkenyl group with for example 2 to 20 carbon atoms; a (hetero)aryl group with for example 1 to 50 carbon atoms; a carboxyl group; an alkyl or aryl carboxylate group with for example 2 to 50 carbon atoms; a formyl group; an alkanoyl or aroyl group with for example 2 to 50 carbon atoms; a carbamoyl group; an N-substituted alkyl or aryl carbamoyl group with for example 2 to 50 carbon atoms; an amino group; an N-substituted alkyl or arylamino group with for example 1 to 50 carbon atoms; a formamido group; an alkyl or aryl amido group with for example 2 to 50 carbon atoms; an alkoxy or aryloxy group with for example 1 to 50 carbon atoms; cyano; nitro; halogen and an alkyl or arylthio group with for example 1 to 50 carbon atoms.

Preferably one of the three substituents R³, R⁴, R⁵, is hydrogen, more preferably two of the three substituents are hydrogen.

Suitable examples of olefins with formula (1) are aliphatic alkenes, for example ethylene, propylene, 1-butene, 2-butene, 1,3-butadiene and 1-decene; acrylic acids, for example acrylic acid, and methacrylic acid; salts of acrylic acids, for example sodium acrylate; acrylate esters, for example methyl acrylate, n-butyl acrylate, t-butyl acrylate, 2-ethyl-hexylacrylate, benzyl acrylate, methyl methacrylate, and n-butyl methacrylate; acrolein; acrolein-dimethyl acetal; olefinic nitriles, for example acrylonitrile and methacrylonitrile; olefinic amides, for example acrylamide and N,N-dimethylacrylamide; maleates, for example di-n-butyl maleate; aromatic alkenes, for example styrene, 2-vinyl naphthalene, 4-vinyl anisole, 4-vinyl aniline, 4-vinyl benzaldehyde, 4-vinyl benzoic acid and 4-vinyl pyridine; olefinic ethers, for example cyclohexyl vinyl ether; 3-butenoic acid; 1-vinyl acetamide; 1-vinyl formamide; vinyl acetate; 1-vinyl-2-pyrrolidone; 1-vinyl-2-caprolactam; vinyl trimethylsilane and sodium salt of vinyl sulphonic acid. Optionally substituted cyclic olefins, for example cyclohexene, indene and cyclooctene, where R³ and R⁴, R³ and R⁵ or R⁴ and R⁵ from formula (1) together with the carbon atoms to which they are bound form a ring structure, also are olefins that can be suitably used in the method according to the invention. Preferably use is made of optionally substituted aliphatic olefins with 2-5 carbon atoms or olefins substituted with a carboxyl group, a carboxyester, a nitrile, an optionally substituted amido group, a nitro group or a halogenated hydrocarbon.

In another embodiment of the invention use is made of an organoboron compound with the formula Ar-B(OR¹) -OR² as second reactant, where Ar stands for an optionally substituted (hetero)aryl group and R¹ and R² each independently represent H or an alkyl group or, together with the O-atoms to which they are bound and the B-atom, form a ring with 2-5 C-atoms. Preferably an optionally substituted (hetero)aryl boric acid or, of course, its anhydride is used. Suitable examples of (hetero)aryl groups and substituents are the same as given above for the (hetero)aromatic bromide compound. Examples of suitable (hetero)aryl boric acids are phenylboric acid and p-tolyl boric acid.

The C-C coupling reaction in the process according to the invention is carried out in the presence of a palladium salt without a ligand. Any arbitrary palladium salt can be used as a catalyst, for example a palladium carboxylate, for example the cheap Pd(OC(O)CH₃)₂ or Pd(OC(O)C₆H₅)₂, a palladium halide, for example PdCl₂, PdBr₂ or Pdl₂, or a sodium palladium halide, for example Na₂PdCl₄ or Na₂PdCl₆.

The ratio between palladium, calculated as the quantity of palladium in the palladium salt, and the optionally substituted (hetero)aromatic bromide compound lies between 0.01 and 0.1 mol% palladium, most preferably between 0.01 and 0.05 mol%.

Suitable solvents that can be used in the process according to the invention are aprotic dipolar solvents, for example dimethyl formamide (DMF), dimethyl acetamide (DMA), 1-methyl pyrrolidinone (NMP), dimethyl sulphoxide (DMSO), acetonitrile or toluene. In specific cases reactants and/or products can serve as a solvent.

The C-C coupling reaction is carried out in the presence of a base in the method according to the invention. Examples of suitable bases are mentioned in for example "Metal-catalyzed Cross-coupling reactions", F. Diederich and P.J. Stang Eds., Wiley-VCH, Weinheim, 1998, Chapters 2 and 3. The base is preferably chosen from the group of tertiary amines, pyridines and alkali metal acetates, alkali metal hydroxides, alkali metal alkoxides, alkali metal phosphates, alkali metal carbonates, and alkali metal hydrogen carbonates. More preferably, the base is chosen from NaOAc, KOAc, K₂CO₃, Na₂CO₃,CaCO₃, K₃PO₄, NaHCO₃ or trialkylamines, in which the alkyl groups each preferably contain, independently of each other, 1 to 20, in particular 1 to 10 carbon atoms, for example triethylamine, tri(n-butyl)amine, methyldiisopropylamine or methyldicyclohexylamine.

The process according to the invention can be used in the presence of one or more additives that are customary in such reactions and that are mentioned in for example "Metal-catalyzed Cross-coupling reactions", F. Diederich and P.J. Stang Eds., Wiley-VCH, Weinheim, 1998, Chapters 2 and 3. Additives that can for example be used are phase-transfer catalysts, for example quaternary ammonium salts, in particular tetrabutylammonium chloride or bromide, triethylbenzylammonium bromide, trioctylbenzylammonium chloride, tetrapropylammonium bromide, or tetraethylammonium chloride, and carbonyl compounds, for example (α) -diketones, in particular 1,2-cyclohexanedione, α- hydroxy ketones, for example 2-hydroxycyclohexanone, aliphatic aldehydes and benzoquinone.

The temperature at which the C-C coupling reaction according to the invention is carried out is not particularly critical. One skilled in the art can simply determine the optimum temperature for his specific reaction system. Preferably the reaction temperature lies between 25 and 250°C, more preferably between 50 and 175°C.

The invention will be elucidated with the following examples.

### Examples

### Definitions

C_{end} = number of moles of product formed at the end of the reaction.
D₀ = number of moles of optionally substituted (hetero)aromatic bromide compound at the start of the reaction.
Dₑ = number of moles of optionally substituted (hetero)aromatic bromide compound at the end of the reaction.
Yield = C_{end}/D₀ * 100%
Conversion = (D₀-Dₑ)D₀ * 100 %
Selectivity = (yield/conversion) * 100 %

### Example I

### C-C coupling reaction of 4-bromoacetophenone and n-butyl acrylate

NaOAc (OAc stands for OC(O)CH₃) (2.46 g, 30 mmol) and 4-bromoacetophenone (3.03 g, 15.2 mmol) were weighed out into a 50 ml Schlenk vessel. After addition of 27 ml 1-methylpyrrolidinone (NMP) and a magnetic stirrer the Schlenk vessel was closed with a rubber septum. Under a nitrogen atmosphere a syringe was then used to add, with stirring: Pd(OAc)₂ (0.4 mg; 0.0018 mmol, 0.012 mol% relative to 4-bromoacetophenone) dissolved in 3 ml NMP and dihexyl ether (328 mg, 1,76 mmol) as internal standard for GC analysis. The reaction mixture was heated. At 100°C n-butyl acrylate (3.78 g, 29.5 mmol) was added with the aid of a syringe. The reaction mixture was heated to the reaction temperature (130°C) and the conversion was monitored by subjecting drawn samples to GC analysis. After 120 minutes the conversion was 95% and the selectivity to the trans-product was 96%.

### Example II

### C-C coupling reaction of 3-bromopyridine and n-butyl acrylate

NaOAc (2.46 g, 30 mmol) was weighed out into a 50 ml Schlenk vessel. After addition of 27 ml NMP and a magnetic stirrer the Schlenk vessel was closed with a rubber septum. Under a nitrogen atmosphere a syringe was then used to add, with stirring: 3-bromopyridine (2.57 g, 16.3 mmol), Pd(OAc)₂ (0.4 mg; 0.0018 mmol, 0.011 mol% relative to 3-bromopyridine) dissolved in 3 ml NMP and dihexyl ether (355.4 mg, 1.91 mmol) as internal standard for GC analysis. The reaction mixture was heated and at 100°C n-butyl acrylate (3.53 g, 26.2 mmol) was added with the aid of a syringe. The reaction mixture was heated to the reaction temperature (130°C) and the conversion was monitored by GC analysis of drawn samples. After 21.5 hours the conversion was 94% and the selectivity 95%.
After cooling the reaction mixture was poured out into 150 ml water and extracted with 80 ml toluene. The aqueous layer was extracted once again with 50 ml toluene. The collected organic layer was washed with water (3 times 125 ml), saturated aqueous NaCl solution (1 time 150 ml), dried with the aid of Na₂SO₄, and filtered through Celite. The Celite phase material was washed with toluene (1 time 150 ml) and the filtrate was thickened with the aid of a film-type evaporator. ¹H NMR of the light-orange liquid (3.6 g) demonstrated the presence of the coupling product and traces of the starting material and dihexyl ether.

### Example III

### C-C coupling reaction of 4-bromoacetophenone and n-butyl acrylate on 2.5 litre-scale

Under nitrogen the following materials were introduced into a double-walled 3 litre reactor: Pd(OAc)₂ (30.4 mg, 0.135 mmol, 0.0099 mol% relative to 4-bromoacetophenone), 2 litres of NMP, 4-bromoacetophenone (272 g, 1.36 mole), and NaOAc (152 g, 1.85 mole). The reaction mixture was heated to 110°C with stirring and at that temperature a start was made with the portionwise addition of n-butyl acrylate (300 g, 2.34 mole, in approximately 1 hour). The mixture was meanwhile heated further to 140°C. Within 30 minutes, after all n-butyl acrylate was added, the conversion was > 99% (GC analysis). After cooling to approximately 80°C the reaction mixture was poured out into 1000 g ice and extracted with 800 ml toluene. The aqueous layer was extracted once again with toluene (3 times 400 ml). The collected organic layer was washed with water (3 times 250 ml), saturated aqueous NaCl solution (2 times 150 ml), dried with the aid of Na₂SO₄, and filtered. The filtrate was thickened to 420 g with the aid of a film-type evaporator and analyzed with the aid of GC. The yield of coupled trans-product amounted to 98%.

### Example IV

### C-C coupling reaction of 3-bromopyridine and n-butyl acrylate on a 2.5 litre scale

Under nitrogen the following materials were introduced into a double-walled 3 litre reactor: Pd(OAc)₂ (152 mg, 0.68 mmol, 0.049 mol% relative to 3-bromopyridine), 2 litres of NMP, 3-bromopyridine (220 g, 1.39 mole) and NaOAc (152 g, 1.85 mole). The reaction mixture was heated to 110° with stirring and at that temperature the portionwise addition of n-butyl acrylate was started (300 g, 2.34 mole, in approximately 1 hour). The mixture was meanwhile heated further to 140°C. After 22 reaction hours the conversion was > 99% (GC analysis). After cooling to approximately 80°C the reaction mixture was poured out into 1000 g ice and extracted with 800 ml toluene. The aqueous layer was extracted once again with toluene (3 times 400 ml). The collected organic layer was washed with water (3 times 250 ml), saturated aqueous NaCl solution (2 times 150 ml), dried with the aid of Na₂SO₄, and filtered. The filtrate was thickened to 409 g with the aid of a film-type evaporator and analyzed with the aid of GC. The yield of coupled trans-product amounted to 87%.

### Example V

### C-C coupling reaction of 3-bromopyridine and t-butyl acrylate

Pd(OAc)₂ (3.5 mg; 0.0156 mmol, 0.044 mol% relative to 3-bromopyridine) and NaOAc (3.8 g, 48.3 mmol) were weighed out into a 100 ml Schlenk vessel. 50 ml NMP and a magnetic stirrer were added and the Schlenk vessel was closed with a rubber septum. Under a nitrogen atmosphere and with stirring 3-bromopyridine (5.51 g, 34.9 mmol) was added with the aid of a syringe. The reaction mixture was heated and at 100°C t-butyl acrylate (7.4 g, 57.8 mmol) was added with the aid of a syringe. The reaction mixture was heated to the reaction temperature (130°C) and the conversion was monitored by GC analysis of drawn samples. After 18 hours the conversion was > 95%.

### Example VI

### C-C-coupling reaction of bromobenzene and sodium acrylate

In a 50 ml Schlenk tube was added: Pd(OAc)₂ (1.6 mg, 0.0071 mmol, 0.041 mol% relative to bromobenzene), bromobenzene (2.7 g, 17.2 mmol), sodium acrylate (2.4 g, 25.6 mmol) and 25 ml of NMP. A magnetic stirrer was added and the Schlenk tube was closed with a rubber stopper. Nitrogen gas was applied and the tube was placed in an oil bath of 135°C and stirred overnight. GC analysis showed a conversion of 86% after 2 hours (98% after 21 hours, no sampling in between) with a selectivity of 98% to the trans-product.

### Example VII

### C-C-coupling reaction of 4-bromoacetophenone and styrene

In a 25 ml vial was added: Pd(OAc)₂ (1.12 mg, 0.0050 mmol, 0.050 mol% relative to 4-bromoacetophenone), 4-bromoacetophenone (1.99 g, 10 mmol), styrene (1.3 g, 12.5 mmol), NaOAc (1.0 g, 12 mmol) and NMP (13 ml). A magnetic stirrer was added and the vial was closed with a plastic cap. The vial was placed in an oil bath of 135°C and stirred magnetically. A sample after 1 hour showed complete conversion. The ratio of trans, cis and methylene product was 94 : 1 : 5, respectively.

### Examples VIII to XXX

### C-C-coupling reaction of several different aryl bromides and several different olefins

In an automated synthesis robot (ASW 2000, from Chemspeed™) 23 double walled reaction vessels were each filled with NaOAc (-2.7 mmol, slight excess relative to the aryl bromide). The vessels were closed and nitrogen gas was applied. To each vessel was added subsequently NMP (1.5 ml), dihexyl ether (0.50 mmol, internal standard for GC), 2.0 ml of a solution of the aryl bromide (1 M in NMP), and 0.25 ml of a solution of Pd(OAc)₂ (0.0035 M in NMP, 0.00089 mmol Pd(OAc)₂ in each vessel = 0.045 mol% compared to the aryl bromide), by using the fully automated syringe of the robot. All vessels were shaken and heated up to 125°C. The different olefins (2.4 mmol) were added (t = 0) and the vessels were further heated to 135°C. After 1, 2, 5, and 15 hours a sample (0.1 ml) of every vessel was taken. The samples were diluted and analysed by GC. The results are shown in Table 1.

**Table 1**

| Arylbromide | Olefin | Time (hr) | Conversion (%, by GC) | Product | Yield (%, by GC) |
|---|---|---|---|---|---|
| 4-bromoacetophenone | *n*-Butylacrylate | 1 | 100 | 3-(4-Acetyl-phenyl)-acrylic acid butyl ester | 99 |
| 4-bromobenzaldehyde | *n*-Butylacrylate | 1 | 100 | 3-(4-Formyl-phenyl)-acrylic acid butyl ester | 100 |
| 2-bromobenzonitrile | *n*-Butylacrylate | 1 | 100 | 3-(2-Cyano-phenyl)-acrylic acid butyl ester | 95 |
| 4-bromobenzonitrile | *n*-Butylacrylate | 1 | 100 | 3-(4-Cyano-phenyl)-acrylic acid butyl ester | 98 |
| 4-bromonitrobenzene | *n*-Butylacrylate | 1 | 100 | 3-(4-Nitro-phenyl)-acrylic acid butyl ester | 95 |
| 2-bromo-6-methoxynaphthalene | *n*-Butylacrylate | 1 | 100 | 3-(6-Methoxy-naphthalen-2-yl)-acrylic acid butyl ester | 96 |
| 4-bromobiphenyl | *n*-Butylacrylate | 2 | 95 | 3-Biphenyl-4-yl-acrylic acid butyl ester | 90 |
| 1-bromonaphthalene | *n*-Butylacrylate | 2 | 100 | 3-Naphthalen-2-yl-acrylic acid butyl ester | 91 |
| 9-bromophenanthrene | *n*-Butylacrylate | 2 | 100 | 3-Phenanthren-9-yl-acrylic acid butyl ester | 93 |
| 4-bromoanisole | *n*-Butylacrylate | 5 | 93 | 3-(4-Methoxy-phenyl)-acrylic acid butyl ester | 94 |
| bromobenzene | *n*-Butylacrylate | 15 | 95 | 3-Phenyl-acrylic acid butyl ester | 90 |
| 3-bromopyridine | *n*-Butylacrylate | 15 | 100 | 3-Pyridin-3-yl-acrylic acid butyl ester | 97 |
| 4-Bromoanisol | *t*-Butylacrylate | 5 | 91 | 3-(4-Methoxy-phenyl)-acrylic acid *tert*-butyl ester | 90 |
| Bromobenzene | *t*-Butylacrylate | 1 | 96 | 3-Phenyl-acrylic acid *tert*-butyl ester | 94 |
| 1-Bromo-2-fluorobenzene | *t*-Butylacrylate | 1 | 100 | 3-(2-Fluoro-phenyl)-acrylic acid fert-butyl ester | 100 |
| 1-Bromo-3-fluorobenzene | *t*-Butylacrylate | 1 | 100 | 3-(3-Fluoro-phenyl)-acrylic acid *tert*-butyl ester | 100 |
| 1-Bromo-4-fluorobenzene | *t*-Butylacrylate | 1 | 100 | 3-(4-Fluoro-phenyl)-acrylic acid *tert*-butyl ester | 100 |
| (4-Bromo-phenyl)-dimethyl-amine | *t*-Butylacrylate | 5 | 88 | 3-(4-Dimethylamino-phenyl)-acrylic acid *tert*-butyl ester | 88 |
| 1-Chloro-4-bromobenzene | Styrene | 15 | 99 | *Trans*-4-chlorostilbene | 92 |
| 2-Bromo-6-methoxynaphthalene | Styrene | 15 | 97 | 2-Methoxy-6-styryl-naphthalene | 94 |
| 4-Bromoacetophenone | But-3-en-2-ol | 15 | 99 | 4-(4-Acetyl-phenyl)-butan-2-one | 92 |
| 1-Chloro-4-bromobenzene | But-3-en-2-ol | 15 | 93 | 4-(4-Chloro-phenyl)-butan-2-one | 88 |
| 2-Bromo-6-methoxynaphthalene | But-3-en-2-ol | 15 | 92 | 4-(6-Methoxy-naphthalen-2-yl)-butan-2-one | 86 |

## Claims

1. Process for carrying out a homogeneously catalyzed C-C coupling reaction between an optionally substituted (hetero)aromatic bromine compound and a second reactant, which is chosen from the group of olefins in which at least one of the substituents on the olefinic Sp² carbon atoms is a hydrogen atom, and the group of organoboron compounds with the formula Ar-B(OR¹) -OR², where Ar stands for an optionally substituted (hetero)aryl group and R¹ and R² each independently represent H or an alkyl group or, together with the O-atoms to which they are bound and the B-atom, form a ring with 2-5 C-atoms, in the presence of a palladium catalyst, an aprotic dipolar solvent and a base, **characterized in that** as palladium catalyst a palladium salt without a ligand is used and **in that** the reaction carried out at a ratio between the quantity of palladium present in the palladium salt and the optionally substituted (hetero)aromatic bromide compound of between 0.01 and 0.1 mol%.

2. Process according to claim 1, **characterized in that** the optionally substituted (hetero)aromatic bromide compound contains at least one hetero atom chosen from N, O and S.

3. Process according to claim 1 or 2, **characterized in that** the second reactant is an optionally substituted, aliphatic olefin with 2-5 carbon atoms.

4. Process according to claim 3, **characterized in that** the second reactant is an olefin substituted with a carboxyl group, a carboxyester, a nitrile group, an optionally substituted amido group, a nitro group or a halogenated hydrocarbon group.

5. Process according to claim 1 or 2, **characterized in that** the second reactant is an (hetero)aryl boric acid or its anhydride.

6. Process according to any one of claims 1-5, **characterized in that** the ratio between the quantity of palladium present in the palladium salt and the optionally substituted (hetero)aromatic bromine compound lies between 0.01 and 0.05 mol%.

7. Process according to any one of claims 1-6, **characterized in that** the aprotic dipolar solvent is chosen from dimethylformamide and N-methylpyrrolidinone.

8. Process according to any one of claims 1-7, **characterized in that** a phase-transfer catalyst is also used.

9. Process according to any one of claims 1-8, **characterized in that** a carbonyl compound, for example an α-diketone, in particular 1,2-cyclohexanedione, an α-hydroxyketone, for example 2-hydroxycyclohexanone, an aliphatic aldehyde or benzoquinone, is also used.

## Patentansprüche

1. Verfahren zur Durchführung einer homogen katalysierten C-C-Kupplungsreaktion zwischen einer gegebenenfalls substituierten (hetero)aromatischen Bromverbindung und einem zweiten Reaktanten, der aus der Gruppe von Olefinen, in denen mindestens einer der Substituenten an den olefinischen sp²-Kohlenstoffatomen ein Wasserstoffatom ist, und der Gruppe von Organoborverbindungen mit der Formel Ar-B(OR¹)-OR², wobei Ar für eine gegebenenfalls substituierte (Hetero)arylgruppe steht und R¹ und R² jeweils unabhängig voneinander für H oder eine Alkylgruppe stehen oder gemeinsam mit den O-Atomen, an die sie gebunden sind, und dem B-Atom einen Ring mit 2-5 C-Atomen bilden, ausgewählt wird, in Gegenwart eines Palladiumkatalysators, eines aprotischen dipolaren Lösungsmittels und einer Base, **dadurch gekennzeichnet, daß** man als Palladiumkatalysator ein Palladiumsalz ohne Ligand verwendet und die Reaktion bei einem Verhältnis zwischen der in dem Palladiumsalz vorliegenden Palladiummenge und der gegebenenfalls substituierten (hetero)aromatischen Bromverbindung zwischen 0,01 und 0,1 Mol-% durchführt.

2. Verfahren nach Anspruch 1, **dadurch gekennzeichnet, daß** die gegebenenfalls substituierte (hetero)-aromatische Bromverbindung mindestens ein unter N, O und 5 ausgewähltes Heteroatom enthält.

3. Verfahren nach Anspruch 1 oder 2, **dadurch gekennzeichnet, daß** es sich bei dem zweiten Reaktanten um ein gegebenenfalls substituiertes, aliphatisches Olefin mit 2-5 Kohlenstoffatomen handelt.

4. Verfahren nach Anspruch 3, **dadurch gekennzeichnet, daß** es sich bei dem zweiten Reaktanten um ein mit einer Carboxylgruppe, einem Carboxyester, einer Nitrilgruppe, einer gegebenenfalls substituierten Amidogruppe, einer Nitrogruppe oder einer halogenierten Kohlenwasserstoffgruppe substituiertes Olefin handelt.

5. Verfahren nach Anspruch 1 oder 2, **dadurch gekennzeichnet, daß** es sich bei dem zweiten Reaktanten um eine (Hetero)arylborsäure oder ihr Anhydrid handelt.

6. Verfahren nach einem der Ansprüche 1-5, **dadurch gekennzeichnet, daß** das Verhältnis zwischen der in dem Palladiumsalz vorliegenden Palladiummenge und der gegebenenfalls substituierten (hetero)aromatischen Bromverbindung zwischen 0,01 und 0,05 Mol-% liegt.

7. Verfahren nach einem der Ansprüche 1-6, **dadurch gekennzeichnet, daß** man das aprotische dipolare Lösungsmittel unter Dimethylformamid und N-Methylpyrrolidon auswählt.

8. Verfahren nach einem der Ansprüche 1-7, **dadurch gekennzeichnet, daß** man auch einen Phasentransferkatalysator verwendet.

9. Verfahren nach einem der Ansprüche 1-8, **dadurch gekennzeichnet, daß** man auch eine Carbonylverbindung, beispielsweise ein α-Diketon, insbesondere 1,2-Cyclohexandion, ein α-Hydroketon, beispielsweise 2-Hydroxycyclohexanon, einen aliphatischen Aldehyd oder Benzochinon verwendet.

## Revendications

1. Procédé de réalisation d'une réaction de couplage C-C à catalyse homogène entre un composé bromé (hétéro)aromatique éventuellement substitué et un deuxième réactif qui est choisi parmi le groupe d'oléfines dans lesquelles au moins l'un des substituants sur les atomes de carbone sp² oléfiniques est un atome d'hydrogène, et le groupe de composés organoborés de formule Ar-B(OR¹)-OR², dans laquelle Ar représente un groupe (hétéro)aryle éventuellement substitué et R¹ et R² représentent chacun indépendamment un atome d'hydrogène ou un groupe alkyle ou, conjointement avec les atomes d'oxygène auxquels ils sont liés et l'atome de bore, forment un noyau renfermant de 2 à 5 atomes de carbone, en présence d'un catalyseur au palladium, d'un solvant dipolaire aprotique et d'une base, **caractérisé en ce que** l'on utilise un sel de palladium sans un ligand en tant que catalyseur au palladium et **en ce que** la réaction est réalisée à un rapport entre la quantité de palladium présent dans le sel de palladium et le composé bromé (hétéro)aromatique éventuellement substitué compris entre 0,01 et 0,1 % en moles.

2. Procédé selon la revendication 1, **caractérisé en ce que** le composé bromé (hétéro)aromatique éventuellement substitué renferme au moins un hétéroatome choisi parmi un atome d'azote, un atome d'oxygène et un atome de soufre.

3. Procédé selon la revendication 1 ou 2, **caractérisé en ce que** le deuxième réactif est une oléfine aliphatique éventuellement substituée, renfermant de 2 à 5 atomes de carbone.

4. Procédé selon la revendication 3, **caractérisé en ce que** le deuxième réactif est une oléfine substituée par un groupe carboxyle, un groupe carboxyester, un groupe nitrile, un groupe amido éventuellement substitué, un groupe nitro ou un groupe hydrocarboné halogéné.

5. Procédé selon la revendication 1 ou 2, **caractérisé en ce que** le deuxième réactif est un acide (hétéro)arylborique ou son anhydride.

6. Procédé selon l'une quelconque des revendications 1 à 5, **caractérisé en ce que** le rapport entre la quantité de palladium présent dans le sel de palladium et le composé bromé (hétéro)aromatique éventuellement substitué est compris entre 0,01 et 0,05 % en moles.

7. Procédé selon l'une quelconque des revendications 1 à 6, **caractérisé en ce que** le solvant dipolaire aprotique est choisi parmi le diméthylformamide et la N-méthylpyrrolidinone.

8. Procédé selon l'une quelconque des revendications 1 à 7, **caractérisé en ce qu'**un catalyseur à transfert de phase est également utilisé.

9. Procédé selon l'une quelconque des revendications 1 à 8, **caractérisé en ce qu'**un composé carbonyle, par exemple, une α-dicétone, en particulier la 1,2-cyclohexanedione, une α-hydroxycétone, par exemple, la 2-hydroxycyclohexanone, un aldéhyde aliphatique ou la benzoquinone, est également utilisé.
